(19) Europäisches Patentamt
European Patent Office
Office européen des brevets

(11) Veröffentlichungsnummer: **0 128 453**
**B1**

(12) # EUROPÄISCHE PATENTSCHRIFT

(45) Veröffentlichungstag der Patentschrift: **11.10.89**

(51) Int. Cl.⁴: **C 07 D209/48**

(21) Anmeldenummer: **84106114.6**

(22) Anmeldetag: **29.05.84**

(54) **Verfahren zur Herstellung von Fluorphthalsäureimiden.**

(30) Priorität: **03.06.83 DE 3320089**

(43) Veröffentlichungstag der Anmeldung: **19.12.84 Patentblatt 84/51**

(45) Bekanntmachung des Hinweises auf die Patenterteilung: **11.10.89 Patentblatt 89/41**

(84) Benannte Vertragsstaaten: **BE CH DE FR GB IT LI**

(56) Entgegenhaltungen:
**EP-A- 0 017 058**
**EP-A- 0 055 630**
**DE-B- 2 334 379**
**US-A- 2 692 267**
**CHEMICAL ABSTRACTS, Band 81, Nr. 6, 12. August 1974, Seite 366, Spalte 2, Zusammenfassungsnr. 30876e, Columbus, Ohio, US; L.R. CASWELL et al.: "Dipole moments of some 3- and 4-substituted phthalimides and phthalic anhydrides. Influence of steric and resonance effects"**

(73) Patentinhaber: **BASF Aktiengesellschaft**
**Carl-Bosch-Strasse 38**
**D-6700 Ludwigshafen (DE)**

(72) Erfinder: **Hamprecht, Gerhard, Dr.**
**Rote-Turm-Strasse 28**
**D-6940 Weinheim (DE)**
Erfinder: **Varwig, Juergen, Dr.**
**Bitterstrasse 21**
**D-6900 Heidelberg (DE)**
Erfinder: **Rohr, Wolfgang, Dr.**
**In der Dreispitz 13**
**D-6706 Wachenheim (DE)**

**EP 0 128 453 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein neues Verfahren zur Herstellung von Fluorphthalsäureimiden durch Umsetzung der entsprechenden Fluorphthalsäureanhydride mit Harnstoff in einem aliphatischen Sulfon.

Es ist aus US-PS 2 692 267 bekannt, daß man substituierte Imino-phthalimidine dadurch herstellen kann, daß man die entsprechenden Phthalsäureanhydride mit Harnstoff in Gegenwart von Ammoniummolybdat oder Ammoniumvanadat als Katalysator umsetzt. Nach den Angaben in J. Org. Chem. 39, 1527 (1974) erhält man 3-Fluorphthalimid durch 3-stündiges Erhitzen eines Gemisches aus 1 Mol 3-Fluorphthalsäureanhydrid und 1,11 Mol Harnstoff in Nitrobenzol auf 170 bis 180 °C. Man erhält dabei ein Rohprodukt, das zur Reinigung zweimal aus Benzol umkristallisiert und dann im Vakuum sublimiert werden muß ; die Ausbeute beträgt 81 %.

Es wurde nun gefunden, daß man Fluorphthalsäureimide der Formel

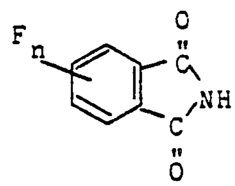

(I)

in der n 1 oder 2 bedeutet, durch Umsetzung von Fluorphthalsäureanhydriden der Formel

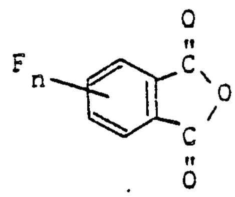

(II)

in der n die vorgenannte Bedeutung besitzt, mit Harnstoff in Gegenwart eines Lösungsmittels bei höherer Temperatur erheblich vorteilhafter herstellen kann, wenn man pro Mol Fluorphthalsäureanhydrid 0,4 bis 0,8 Mol, vorzugsweise 0,5 bis 0,6 Mol Harnstoff und als Lösungsmittel ein aliphatisches Sulfon einsetzt, und die Umsetzung zuerst bei einer Temperatur von 110 bis 140 °C und nach dem Ende der Gasabspaltung bei einer Temperatur von 150 bis 170 °C durchführt.

Fluorphthalsäureanhydride der Formel II sind beispielsweise 3-Fluorphthalsäureanhydrid, 4-Fluorphthalsäureanhydrid, 3,6-Difluorphthalsäureanhydrid, 4,5-Difluorphthalsäureanhydrid und 3,5-Difluorphthalsäureanhydrid.

Als aliphatische Sulfone sind z. B. die Verbindungen der Formel

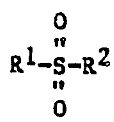

(III)

geeignet, in der $R^1$ und $R^2$ gleich oder verschieden sind und jeweils einen aliphatischen Rest, vorzugsweise einen Alkylrest mit 1 bis 8, insbesondere 1 bis 4 Kohlenstoffatomen oder $R^1$ und $R^2$ zusammen einen Alkylenrest mit 4 oder 5 Kohlenstoffatomen bedeuten. Geeignete Lösungsmittel der genannten Art sind beispielsweise Dimethylsulfon, Diethylsulfon, Dipropylsulfon, Diisopropylsulfon, Dibutylsulfon, Diisobutylsulfon, Methylethylsulfon, Tetramethylensulfon (= Sulfolan) und Pentamethylensulfon, von denen Sulfolan bevorzugt ist. Zweckmäßig verwendet man das Lösungsmittel in einer Menge von 50 bis 1 000 Gewichtsprozent, vorzugsweise 100 bis 300 Gewichtsprozent, bezogen auf Ausgangsstoff II.

Man nimmt die Umsetzung in zwei Stufen vor, wobei man die erste Stufe bei einer Temperatur von 110 bis 140 °C, insbesondere bei 120 bis 135 °C und die zweite Stufe bei einer Temperatur von 150 bis 170 °C, insbesondere bei 160 bis 170 °C durchführt. Die erste Stufe der Umsetzung ist mit dem Ende der Gasabspaltung beendet. Sie benötigt ungefähr 30 bis 240 Minuten. Die zweite Stufe der Umsetzung ist nach etwa 10 bis 20 Minuten beendet. Der Ausgangsstoff II, das Sulfon III und der Harnstoff können in beliebiger Reihenfolge im angegebenen Temperaturbereich miteinander vermischt werden. Zweckmäßig vermischt man jedoch zunächst bei 30 bis 40 °C den Ausgangsstoff II mit dem Sulfon, fügt dann unter Rühren den Harnstoff hinzu und erhitzt das Gemisch in dem angegebenen Temperaturbereich. Man erhitzt bis keine Gasentwicklung mehr beobachtet wird. Man kann dabei drucklos oder unter Druck, kontinuierlich oder diskontinuierlich arbeiten. Der Harnstoff wird z. B. in einer Menge von 0,4 bis 0,8,

vorzugsweise 0,5 bis 0,6, insbesondere 0,51 bis 0,55 Mol, pro Mol Ausgangsstoff II angewendet. Man verfährt z. B. folgendermaßen : Zu dem Gemisch des Ausgangsstoffes II in dem Sulfon gibt man den Harnstoff und hält die durch Verrühren erhaltene Mischung 1/2 bis 4 Stunden bei der Temperatur der Hauptreaktion bis zum Ende der Gasabspaltung und rührt dann noch 10 bis 20 Minuten bei der Temperatur der Nachreaktion.

Aus dem Reaktionsgemisch trennt man das Fluorphthalsäureimid auf an sich übliche Weise ab, z. B. durch Filtration, Waschen des Festgutes und Destillation von Filtrat und Waschfiltrat.

Nach einer besonders vorteilhaften Ausführungsform des erfindungsgemäßen Verfahrens verwendet man anstelle des Fluorphthalsäureanhydrids der Formel II ein Reaktionsgemisch, das man durch Behandlung des entsprechenden Chlorphthalsäureanhydrids mit einem Säurechlorid der schwefligen Säure oder der Kohlensäure in Gegenwart eines aliphatischen Sulfons und anschließende Umsetzung des Gemisches mit Kaliumfluorid erhält. Unter dem entsprechenden Chlorphthalsäureanhydrid wird die Verbindung der Formel II verstanden, in der F durch Cl ersetzt ist.

Reaktionsgemische der genannten Art werden z. B. erhalten, wenn man das Chlorphthalsäureanhydrid in einer ersten Verfahrensstufe bei Temperaturen bis 150 °C mit einem Säurechlorid der schwefligen Säure oder der Kohlensäure in Gegenwart eines aliphatischen Sulfons behandelt und das Behandlungsgemisch dann in einer zweiten Stufe bei Temperaturen von 150 bis 250 °C mit dem Kaliumfluorid umsetzt. Als Säurechlorid der schwefligen Säure oder der Kohlensäure verwendet man z. B. Thionylchlorid oder Phosgen. Die Säurechloride werden z. B. in einer Menge von 1 bis 20 Gewichtsprozent, bezogen auf das Sulfon, eingesetzt. Als aliphatische Sulfone kommen Verbindungen der Formel III in Betracht. Man verwendet das Sulfon in einer Menge von 50 bis 1 000 Gewichtsprozent, bezogen auf das Chlorphthalsäureanhydrid.

Zweckmäßig ist z. B. die folgende Arbeitsweise : Unter Rühren vermischt man bei 30 bis 40 °C das Chlorphthalsäureanhydrid mit dem Sulfon und gegebenenfalls mit einem N,N-disubstituierten Carbonamid als Katalysator, fügt dann das Säurechlorid hinzu und erhitzt zweckmäßig auf eine Temperatur zwischen 50 und 120 °C, insbesondere auf 70 bis 100 °C. Die erste Stufe ist mit dem Ende der Gasentwicklung beendet. Überschüssiges Säurechlorid kann dann z. B. durch Einblasen eines Inertgases, wie Stickstoff, oder durch Anlegen von Vakuum entfernt werden. Nun setzt man das Kaliumfluorid und gegebenenfalls einen Kronenether oder Cryptanden als Katalysator hinzu und rührt während 1 bis 10 Stunden bei Temperaturen von 150 bis 250 °C, insbesondere von 170 bis 240 °C, bevorzugt von 190 bis 220 °C. Anschließend befreit man das Reaktionsgemisch durch Absaugen oder Abdestillieren vom anorganischen Rückstand, wobei bei der Verwendung von Sulfolan als Lösungsmittel dieses in der Regel zusammen mit dem Fluorphthalsäureanhydrid der Formel II in einem ähnlichen Siedebereich überdestilliert. Zu dem so erhaltenen vom anorganischem Rückstand befreitem Gemisch von Verbindung II und Sulfon gibt man nun unter Rühren den Harnstoff hinzu und führt die erfindungsgemäße Umsetzung wie beschrieben durch. Man kann aber auch direkt das Reaktionsgemisch mit Harnstoff versetzen und die erfindungsgemäße Umsetzung ohne vorherige Abtrennung der anorganischen Nebenprodukte durchführen.

Als N,N-disubstituierte Carbonamide sind solche mit 3 bis 10 Kohlenstoffatomen geeignet, wie N,N-Dimethylformamid, N,N-Dimethylacetamid, N,N-Diethylformamid, N,N-Di-n-propylacetamid, N-Methyl-N-ethyl-formamid oder N,N-Di-isopropylacetamid. Der Katalysator wird zweckmäßig in einer Menge von 0,2 bis 2 Gewichtsprozent, bezogen auf das Säurechlorid, eingesetzt. Kronenether oder Cryptanden sind organische Komplexliganden, die gut zur Bindung von Alkali dienen. Kronenether sind cyclisch angeordnete, neutrale Ethylenglykolether. Die Cryptanden liefen eine dreidimensionale Umhüllung. Bezüglich Herstellung dieser Stoffe, siehe « Kontakte » (1977), Seiten 11 bis 31 und 36 bis 48.

Im Vergleich zu den bekannten Verfahren liefert das Verfahren nach der Erfindung ein erheblich verbessertes Ergebnis in bezug auf Ausbeute und Reinheit der Fluorphthalsäureimide. Eine Isolierung der als Ausgangsmaterial verwendeten Fluorphthalsäureanhydride ist nicht erforderlich, die Gesamtreaktionszeit ist kürzer, Reinigungsprozesse durch Umkristallisation mit organischen Lösungsmitteln oder Sublimation entfallen. Auch ist die Aufarbeitung des Reaktionsgemisches, gerade im Hinblick auf den Umweltschutz, einfacher und betriebssicherer, da außer stöchiometrischen Mengen Kohlendioxid keine Nebenprodukte entstehen. Alle diese vorteilhaften Eigenschaften sind im Hinblick auf den Stand der Technik überraschend.

Die nach dem Verfahren der Erfindung herstellbaren Verbindungen sind wertvolle Ausgangsstoffe für die Herstellung von Pflanzenschutzmitteln, Farbstoffen und Pharmazeutika. So kann man aus ihnen z. B. durch Einwirkung von Natriumhypochlorit unter den Bedingungen des Hofmann-Abbaues isomere 3-Fluor- und 6-Fluoranthranilsäuren herstellen, die man nach Abtrennung des 3-Fluorderivats, mit Benzoychlorid, z. B. nach dem in der DE-PS 2 914 915 beschriebenen Verfahren in das 5-Fluor-2-phenyl-4H-3,1-benzoxazin(4)on überführen kann, das als Pflanzenschutzmittel verwendet wird.

Die in den folgenden Beispielen aufgeführten Teile bedeuten Gewichtsteile :

## Beispiel 1

Eine Mischung von 49,8 Teilen 3-Fluorphthalsäureanhydrid (0,3 Mol) und 9,9 Teilen Harnstoff (0,165 Mol) in 126 Teilen Sulfolan wird unter Rühren auf 125 bis 130 °C erhitzt. Man rührt 30 Minuten bis zum

Ende der Kohlendioxidabspaltung und rührt dann noch weitere 15 Minuten bei 168 °C nach. Anschließend wird das Lösungsmittel bei einer Badtemperatur von 130 bis 160 °C/0,3 mbar abdestilliert (98 Teile). Nach dem Gaschromatogramm enthält es 2,75 Teile 3-Fluorphthalsäureimid. Der Destillationsrückstand wird mit Wasser verrieben, abgesaugt, gewaschen und getrocknet, wobei 44,5 Teile 3-Fluorphthalsäureimid vom Fp 179 bis 182 °C erhalten werden. Die Gesamtausbeute beträgt 47,25 Teile (95,3 % d. Th.) 3-Fluorphthalsäureimid.

Beispiel 2

500 Teile 3-Chlorphthalsäureanhydrid (= 2,739 Mol) und 49,9 Teile Thionylchlorid werden unter Rühren zu 820 Teilen Sulfolan gegeben. Man rührt 30 Minuten bei 100 °C bis zum Ende der Gasentwicklung. Nach dem Abziehen überschüssigen Thionylchlorids im Wasserstrahlvakuum gibt man 175 Teile Kaliumfluorid hinzu und rührt 6 Stunden bei 210 °C. Nach dem Abkühlen auf 60 °C werden 82 Teile Harnstoff (= 1,37 Mol) zugegeben. Man rührt 1 1/2 Stunden bei 130 °C bis zum Ende der Gasabspaltung und rührt dann noch weitere 15 Minuten bei 170 °C. Nun kühlt man auf Raumtemperatur ab, gibt 300 Teile Aceton unter Rühren hinzu und saugt den überwiegend anorganischen Niederschlag ab. Das Filtrat wird am Rotationsverdampfer vom Aceton befreit und der Rückstand anschließend über eine 10 cm Füllkörperkolonne bei einer Badtemperatur von 140 bis 170 °C/0,4 mbar destilliert. Der Destillationsrückstand wird mit Wasser verrührt, abgesaugt und nochmals mit Methyl-tert.-butylether gewaschen. Nach dem Trocknen erhält man 382 Teile (84,5 % d. Th.) 3-Fluorphthalsäureimid vom Fp. 176 bis 179 °C. Durch Behandeln des anorganischen Rückstandes mit 300 Teilen Aceton, Abtrennen und Einengen im Vakuum isoliert man weitere 29 Teile (6,8 % d. Th.) 3-Fluorphthalsäureimid vom Fp. 166 bis 178 °C.

Beispiel 3

Eine Lösung von 250 Teilen 4-Chlorphthalsäureanhydrid (= 1,369 Mol) in 450 Teilen Sulfolan wird während 40 Minuten unter Rühren bei 100 °C mit 50 Teilen Phosgen begast. Nach dem Abziehen überschüssigen Phosgens im Wasserstrahlvakuum gibt man bei 80 °C 95,4 Teile Kaliumfluorid hinzu und rührt 3 1/2 Stunden bei 215 °C. Nun werden Reaktionsprodukt und Sulfolan gemeinsam bei 115 bis 125 °C/0,3 mbar von dem verbleibenden Rückstand abdestilliert. Zum Schluß gibt man zur azeotropen Nachdestillation von nicht übergegangenen 4-Fluorphthalsäureanhydrids nochmals 126 Teile Sulfolan hinzu. Das so erhaltene Destillat (753 Teile) enthält 177 Teile (78 % d. Th.) gaschromatographisch reines 4-Fluorphthalsäureanhydrid.

185 Teile des Destillates (enthaltend 43,4 Teile 4-Fluorphthalsäureanhydrid = 0,261 Mol) werden mit 8,2 Teilen Harnstoff (0,137 Mol) versetzt. Man rührt das Gemisch 3 Stunden bei 135 bis 140 °C bis zum Ende der Gasentwicklung. Zum Schluß erhitzt man noch 10 Minuten auf 160 °C. Das Lösungsmittel wird bei 115 bis 130 °C/0,3 mbar abdestilliert (144,2 Teile). Es enthält nach der gaschromatographischen Untersuchung 9,6 Teile 4-Fluorphthalsäureimid. Der Destillationsrückstand wird mit Wasser verrührt, abgesaugt, gewaschen und getrocknet, wobei 31 Teile 4-Fluorphthalsäureimid vom Fp. 174 bis 176 °C erhalten werden. Die Gesamtausbeute beträgt 40,6 Teile (94,2 % d. Th.) 4-Fluorphthalsäureimid.

**Patentansprüche**

1. Verfahren zur Herstellung von Fluorphthalsäureimiden der Formel

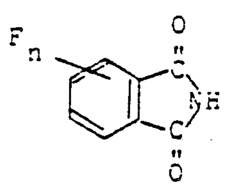

(I)

in der n 1 oder 2 bedeutet, durch Umsetzung von Fluorphthalsäureanhydriden der Formel

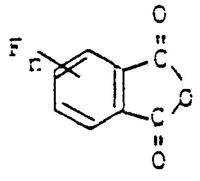

(II)

in der n die vorgenannte Bedeutung hat, mit Harnstoff in Gegenwart eines Lösungsmittels bei höherer Temperatur, dadurch gekennzeichnet, daß man pro Mol Fluorphthalsäureanhydrid 0,4 bis 0,8 Mol, vorzugsweise 0,5 bis 0,6 Mol Harnstoff und als Lösungsmittel ein aliphatisches Sulfon einsetzt, und die Umsetzung zuerst bei einer Temperatur von 110 bis 140 °C und nach dem Ende der Gasabspaltung bei einer Temperatur von 150 bis 170 °C durchführt.

2. Verfahren nach Anspruch 1, dadurch gekennzeichnet, daß man anstelle des Fluorphthalsäureanhydris ein Reaktionsgemisch verwendet, das man durch Behandlung des entsprechenden Chlorphthalsäureanhydrids mit einem Säurechlorid der schwefligen Säure oder der Kohlensäure in Gegenwart eines aliphatischen Sulfons und anschließende Umsetzung des Gemisches mit Kaliumfluorid erhält.

**Claims**

1. A process for the preparation of a fluorophthalimide of the formula

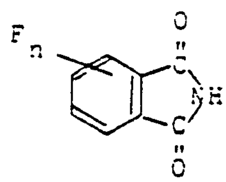

(I)

where n is 1 or 2, by reacting a fluorophthalic anhydride of the formula

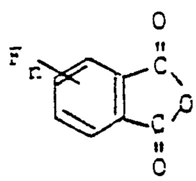

(II)

where n has the above meanings, with urea in the presence of a solvent at elevated temperatures, wherein from 0.4 to 0.8, preferably from 0.5 to 0.6, mole of urea is used per mole of fluorophthalic anhydride, an aliphatic sulfone is employed as the solvent, and the reaction is carried out first at 110-140 °C and, after the evolution of gas has ended, at 150-170 °C.

2. A process as claimed in claim 1, wherein the fluorophthalic anhydride is replaced by a reaction mixture which is obtained by treating the corresponding chlorophthalio anhydride with an acid chloride of sulfurous acid or of carbonic acid in the presence of an aliphatic sulfone, and then reacting the mixture with potassium fluoride.

**Revendications**

1. Procédé de préparation d'imides d'acides fluorophtaliques de la formule

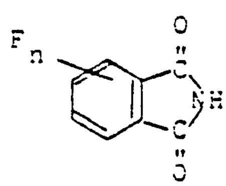

(I)

dans laquelle n est égal à 1 ou à 2, par la réaction d'anhydrides fluorophtaliques de la formule

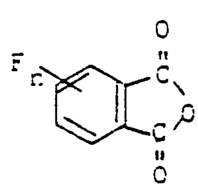

(II)

dans laquelle n possède les significations qui lui ont été attribuées ci-dessus, sur l'urée, en présence d'un solvant, à température élevée, caractérisé en ce que l'on met en œuvre, par mole d'anhydride fluorophtalique, de 0,4 à 0,8 mole, de préférence de 0,5 à 0,6 mole, d'urée et, à titre de solvant, une sulfone aliphatique et on réalise la réaction d'abord à une température de 100 à 140 °C et, après la fin de la séparation ou du dégagement de gaz, à une température de 150 à 170 °C.

2. Procédé suivant la revendication 1, caractérisé en ce qu'au lieu de l'anhydride fluorophtalique, on utilise un mélange réactionnel que l'on obtient par le traitement de l'anhydride chlorophtalique correspondant par un chlorure d'acide de l'acide sulfureux ou de l'acide carbonique, en présence d'une sulfone aliphatique et réaction subséquente du mélange avec du fluorure de potassium.